# FASCICULE DE BREVET EUROPEEN

(11) **EP 0 809 705 B1**
(45) Date de publication et mention de la délivrance du brevet: **21.12.2005**
(21) Numéro de dépôt: 96904146.6
(22) Date de dépôt: 15.02.1996
(51) Int. Cl.: C12N 15/87, A61K 48/00

(54) **COMPOSITIONS CONTENANT DES ACIDES NUCLEIQUES, PREPARATION ET UTILISATION**
NUKLEINSÄURE ENTHALTENDE ZUSAMMENSETZUNG, HERSTELLUNG UND VERWENDUNG
NUCLEIC ACID-CONTAINING COMPOSITION, PREPARATION AND USE THEREOF

(30) Priorité: 17.02.1995 FR 9501865
(43) Date de publication de la demande: 03.12.1997
(73) Titulaire: Aventis Pharma S.A., 92160 Antony (FR)
(72) Inventeur: BYK, Gerardo, F-94000 Créteil (FR); SCHERMAN, Daniel, F-75645 Paris Cédex 13 (FR); SCHWARTZ, Bertrand, F-94700 Maisons-Alfort (FR)
(74) Mandataire: Peaucelle, Chantal
(86) Numéro de dépôt international: PCT/FR1996/000248
(87) Numéro de publication internationale: WO 1996/025508

(56) Documents cités:
- EP-A- 0 388 758
- WO-A-91/17773
- WO-A-93/19768
- BIOCHIMICA AND BIOPHYSICA ACTA, vol. 950, no. 2, 13 Juillet 1988, pages 221-228, XP002008071 BOETTGER, M. ET AL.: "Condensation of vector DNA by the chromosomal protein HMG1 results in efficient transfection"
- DNA, vol. 6, no. 1, Février 1987, pages 81-89, XP002008072 WIERNHUES, U. ET AL.: "A novel method for transfection and expression of reconstituted DNA-protein complexes in eukaryotic cells"
- JOURNAL OF VIROLOGICAL METHODS, vol. 23, no. 2, Février 1989, pages 187-194, XP002008073 CORNETTA, K. & ANDERSON, W.F.: "Protamine sulfate as an effective alternative to polybrene in retroviral-mediated gene transfer: implications for human gene therapy"
- PROTOPLASMA, vol. 96, 1978, pages 209-223, XP002008074 DUBES, G.R. & WEGRZYN, R.J.: "Rapid ephemeral cell sensitization as the mechanism of histone-induced and protamine-induced enhancement of transfection by poliovirus RNA"
- CHEN ET AL: "GALACTOSYLATED HISTONE-MEDIATED GENE TRANSFER AND EXPRESSION" HUMAN GENE THERAPY, vol. 5, 1994, pages 429-435,

### LISTE DE SEQUENCES

(1) INFORMATIONS GENERALES:
   (i) DEPOSANT:
      (A) NOM: RHONE POULENC RORER S.A.
      (B) RUE: 20, Avenue Raymond Aron
      (C) VILLE: ANTONY
      (E) PAYS: FRANCE
      (F) CODE POSTAL: 92165
      (G) TELEPHONE: 40.91.69.22
      (H) TELECOPIE: (1) 40.91.72.96
   (ii) TITRE DE L'INVENTION: COMPOSITION CONTENANT DES ACIDES NUCLEIQUES,PREPARATION ET UTILISATION
   (iii) NOMBRE DE SEQUENCES:9
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 9 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 1:
   Lys Thr Pro Lys Lys Ala Lys Lys Pro
   5
(2) INFORMATIONS POUR LA SEQ ID NO: 2:
   (i) CARACTERISTIQUES DE LA SEQUENCE:
      (A) LONGUEUR: 8 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Ala Thr Pro Ala Lys Lys Ala Ala
   5
(2) INFORMATIONS POUR LA SEQ ID NO: 3:
   (i) CARACTERISTIQUES DE LA SEQUENCE:
      (A) LONGUEUR: 16 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
   Ala Thr Pro Ala Lys Lys Ala Ala Ala Thr Pro Ala Lys Lys Ala Ala
   5 10 15
(2) INFORMATIONS POUR LA SEQ ID NO: 4:
   (i) CARACTERISTIQUES DE LA SEQUENCE:
      (A) LONGUEUR: 18 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
   Lys Thr Pro Lys Lys Ala Lys Lys Pro Lys Thr Pro Lys Lys Ala Lys
   5 10 15
   Lys Pro
(2) INFORMATIONS POUR LA SEQ ID NO: 5:
   (i) CARACTERISTIQUES DE LA SEQUENCE:
      (A) LONGUEUR: 17 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 5:
   Ala Thr Pro Lys Lys Ser Ala Lys Lys Thr Pro Lys Lys Ala Lys Lys Pro
   5 10 15
(2) INFORMATIONS POUR LA SEQ ID NO: 6:
   (i) CARACTERISTIQUES DE LA SEQUENCE:
      (A) LONGUEUR: 26 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Lys Lys Ala Lys Ser Pro Lys Lys Ala Lys Ala Ala Lys Pro Lys Lys Ala
   5 10 15
   Pro Lys Ser Pro Ala Lys Ala Lys Ala
   20 25
(2) INFORMATIONS POUR LA SEQ ID NO: 7:
   (i) CARACTERISTIQUES DE LA SEQUENCE:
      (A) LONGUEUR: 18 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 7:
   Ser Arg Ser Arg Tyr Tyr Arg Gln Arg Gln Arg Ser Arg Arg Arg Arg Arg
   5 10 15
   Arg
(2) INFORMATIONS POUR LA SEQ ID NO: 8:
   (i) CARACTERISTIQUES DE LA SEQUENCE:
      (A) LONGUEUR: 21 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Arg Arg Arg Leu His Arg Ile His Arg Arg Gln His Arg Ser Cys Arg Arg
   5 10 15
   Arg Lys Arg Arg
   20
(2) INFORMATIONS POUR LA SEQ ID NO: 9:
   (i) CARACTERISTIQUES DE LA SEQUENCE:
      (A) LONGUEUR: 26 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 9:
   Pro Lys Lys Lys Arg Lys Val Ala Lys Thr Pro Lys Lys Ala Lys Lys Pro
   5 10 15
   Lys Thr Pro Lys Lys Ala Lys Lys Pro
   20 25

## Description

La présente invention concerne le domaine de la thérapie génique et s'intéresse plus particulièrement au transfert in vitro, ex vivo et/ou in vivo du matériel génétique. Elle propose notamment une nouvelle composition pharmaceutique utile pour transfecter efficacement des cellules. Elle se rapporte également aux utilisations de cette composition.

Des déficiences et/ou anomalies (mutation, expression aberrante, etc) chromosomiques sont à l'origine de nombreuses maladies, à caractère héréditaire ou non. Pendant longtemps, la médecine conventionnelle est demeurée impuissante à leur égard. Aujourd'hui, avec le développement de la thérapie génique, on espère pouvoir désormais corriger ou prévenir ce type d'aberration chromosomique. Cette nouvelle médication consiste à introduire une information génétique, dans la cellule ou l'organe affecté, en vu de corriger cette déficience ou anomalie ou encore, d'y exprimer une protéine d'intérêt thérapeutique.

L'obstacle majeur à la pénétration d'un acide nucléique dans une cellule ou un organe cible, repose sur la taille et la nature polyanionique de cet acide nucléique qui s'opposent à son passage à travers les membranes cellulaires.

Pour lever cette difficulté, diverses techniques sont aujourd'hui proposées dont plus particulièrement la transfection d'ADN nu à travers la membrane plasmique in vivo (WO90/11092) et la transfection d'ADN via un vecteur de transfection.

En ce qui concerne, la transfeetion d'ADN nu, son efficacité demeure encore très faible. Les acides nucléiques nus possèdent une demi vie plasmatique courte en raison de leur dégradation par les enzymes et de leur élimination par les voies urinaires.

Pour ce qui est de la seconde technique, elle propose principalement deux stratégies:
La première met en oeuvre les vecteurs de transfection naturels que sont les virus. Il est ainsi proposé d'utiliser les adénovirus, les herpès, virus, les rétrovirus et plus récemment les virus adéno associés. Ces vecteurs s'avèrent performants sur le plan de la transfection mais on ne peut malheureusement exclure totalement à leur égard certains risques de pathogénicité, réplication et/ou immunogénicité, inhérents à leur nature virale.
La seconde stratégie consiste avantageusement à utiliser des agents non viraux capables de promouvoir le transfert et l'expression d'ADN dans des cellules eucaryotes.

L'objet de la présente invention s'inscrit plus particulièrement dans cette seconde stratégie.

Les vecteurs chimiques ou biochimiques représentent une alternative avantageuse aux virus naturels en particulier pour cette absence de réponse immunologique et/ou recombinaison virale. Ils ne possèdent pas de pouvoir pathogène, le risque de multiplication de l'ADN au sein de ces vecteurs est nul et il ne leur est attaché aucune limite théorique en ce qui concerne la taille de l'ADN à transfecter.

Ces vecteurs synthétiques ont deux fonctions principales, condenser l'ADN à transfecter et promouvoir sa fixation cellulaire ainsi que son passage à travers la membrane plasmique et, le cas échéant, les deux membranes nucléaires.

De part sa nature polyanionique, l'ADN n'a naturellement aucune affinité pour la membrane plasmique des cellules également polyanionique. Pour pallier à cet inconvénient, les vecteurs non viraux possèdent généralement tous des charges polycationiques.

Parmi les vecteurs synthétiques développés, les polymères cationiques de type polylysine et DEAE dextran ou encore les lipides cationiques ou lipofectants sont les plus avantageux. Ils possèdent la propriété de condenser l'ADN et de promouvoir son association avec la membrane cellulaire. Plus récemment, il a été développé le concept de la transfection ciblée, médiée par un récepteur. Cette technique met à profit le principe de condenser l'ADN, grâce au polymère cationique, tout en dirigeant la fixation du complexe à la membrane à l'aide d'un couplage chimique entre le polymère cationique et le ligand d'un récepteur membranaire, présent à la surface du type cellulaire que l'on veut greffer. Les ciblages du récepteur à la transferrine, à l'insuline ou du récepteur des asialoglycoprotéines des hépatocytes ont ainsi été décrits.

Toutefois, les vecteurs synthétiques proposés aujourd'hui sont encore loin d'être aussi performants que les vecteur viraux. Ceci pourrait être la conséquence d'une condensation insuffisante de l'ADN à tranfecter et/ou des difficultés rencontrées par l'ADN transfecté pour sortir de l'endosome et pénétrer dans le noyau cellulaire. Enfin, d'autres inconvenients sont directement associés à la nature des polymères cationiques des lipofectants utilisés.

La présente invention a précisément pour objectif de proposer une solution avantageuse à ces problèmes.

La présente invention se rapporte à une composition pharmaceutique utile pour la transfection d'un acide nucléique caractérisée en ce qu'elle contient outre ledit acide nucléique, au moins un agent de transfection et un composé intervenant au niveau de la condensation dudit acide nucléique, ledit composé dérivant en tout ou partie d'une histone, d'une nucléoline, d'une protamine et/ou de l'un de leurs dérivés.

La demande WO 91/17773 décrit une composition pharmaceutique contenant un acide nucléique, un agent de transfection et un composé intervenant au niveau de la condensation dudit acide nucléique à savoir une histone. Par ailleurs, l'article par Chen et al, Human Gene Therapy, 1994, Vol. 5 pp. 429-435 divulgue l'utilisation de l'histone H1 pour la transfection.

L'invention propose l'utilisation dans des compositions pharmaceutiques de nouveaux composés intervenant au niveau de la condensation d'un acice nucléique constitués de motifs peptidiques ou d'oligopeptides spécifiques.

Plus précisément, l'invention propose une composition pharmaceutique utile pour la transfection d'un acide nucléique contenant outre ledit acide nucléique, un agent de transfection et au moins un composé intervenant au niveau de la condensation dudit acide nucléique caractérisée en ce que ledit composé est constitué en tout ou partie, de motifs peptidiques (KTPKKAKKP) et/ou (ATPAKKAA) répétés de manière continue ou non, le nombre des motifs pouvant varier entre 2 et 10 ou est représenté par un oligopeptide choisi parmi SRSRYYRQRQRSRRRRRR(COOH) et RRRLHRIHRRQHRSCRRRKRR(COOH).

De manière inattendue, la demanderesse a démontré que la présence de tels composés, au sein d'une composition transfectante à base d'un agent de transfection classique, permettait de diminuer considérablement la quantité en cet agent, avec les conséquences bénéfiques qui en découlent sur le plan toxicologique, sans porter un préjudice quelconque à l'activité transfectante de ladite composition. Au contraire, celle-ci possède avantageusement un niveau de transfection supérieur.

Au sens de l'invention, un composé intervenant au niveau de la condensation de l'acide nucléique, couvre tout composé compactant, directement ou non, l'acide nucléique. Plus précisément, ce composé peut soit agir directement au niveau de l'acide nucléique à transfecter soit intervenir au niveau d'un composé annexe qui lui est directement impliqué dans la condensation de cet acide nucléique. De préférence, il agit directement au niveau de l'acide nucléique.

Avantageusement, les motifs peptidiques peuvent être séparés par des liens de nature biochimique, par exemple un ou plusieurs acides aminés.

Le choix particulier à titre de composé selon l'invention, d'un peptide ou pseudopeptide possédant une majorité d'acides aminés à caractère basique comme la lysine, l'histidine ou l'arginine est particulièrement avantageux dans le cadre de la présente invention, Ce composé peut en outre posséder une structure conformationnelle feuillet b. Les acides aminés basiques sont en effet plus spécifiquement impliqués dans les liaisons peptide-acide nucléique. Ils participent à l'établissement des liaisons ioniques hydrogène entre les deux entités favorisant ainsi la condensation de l'acide nucléique. Quant à la structure feuillet β, elle se caractérise par une meilleure accessibilité d'une majorité des liaisons carbonyles et des atomes d'hydrogène qui de part leurs caractères accepteur et donneur respectifs privilégient également la formation de liaisons avec l'acide nucléique à compacter.

Il s'agit plus préférentiellement, de tout ou partie d'une histone, d'une nucléoline, de la protamine et/ou un de leurs dérivés.

Les histones et les protamines sont des protéines cationiques compactant naturellement l'ADN. Elles sont ainsi responsables in vivo de la condensation de l'ADN non transcrit, de l'ADN de certains virus. En ce qui concerne la nucléoline, il s'agit d'une protéine nucléolaire qui semble posséder un effet synergique vis à vis de l'histone H1 lors de la condensation de l'ADN par celle-ci. Dans le cadre de la présente invention, le composé peut être avantageusement représenté par une séquence peptidique dérivant de la partie N terminale de la nucléoline, et plus précisément correspondant à la séquence (ATPAKKAA)₂(COOH) (SEQ ID N°3).

De préférence, le composé mis en oeuvre selon l'invention est une séquence dérivée de l'histone H1, correspondant à la séquence (KTPKKAKKP)₂(COOH). (SEQ ID N°4).

A titre illustratif de cette famille de composés selon l'invention on peut également citer les oligopeptides suivants: et

En ce qui concerne plus particulièrement les séquences dérivant de la protamines pouvant être également mises en oeuvre dans le cadre de la présente invention, on peut notamment proposer les oligopeptides suivants: et

Au sens de la présente invention, le terme dérivé désigne tout peptide, pseudopeptide (peptide incorporant des éléments non biochimiques) ou protéine différant de la protéine ou peptide considéré, obtenu par une ou plusieurs modifications de nature génétique et/ou chimique. Par modification de nature génétique et/ou chimique, on peut entendre toute mutation, substitution, délétion, addition et/ou modification d'un ou plusieurs résidus de la protéine considérée. Plus précisément, par modification chimique, on entend toute modification du peptide ou protéine générée par réaction chimique ou par greffage chimique de molécule(s), biologique(s) ou non, sur un nombre quelconque de résidus de la protéine. Par modification génétique, on entend toute séquence peptidique dont l'ADN hybride avec ces séquences ou des fragments de celles-ci et dont le produit possède l'activité indiquée. De tels dérivés peuvent être générés dans des buts différents, tels que notamment celui d'augmenter l'affinité du polypeptide correspondant pour son(ses) ligand(s), celui d'améliorer ses niveaux de production, celui d'augmenter sa résistance à des protéases, celui d'augmenter et/ou de modifier son activité, ou celui de lui conférer de nouvelles propriétés pharmacocinétiques et/ou biologiques. Parmi les dérivés résultant d'une addition, on peut citer par exemple les séquences peptidiques chimères comportant une partie hétérologue supplémentaire liée à une extrémité. Le terme dérivé comprend également les séquences protéiques homologues à la séquence considérée, issues d'autres source cellulaires et notamment de cellules d'origine humaine, ou d'autres organismes, et possédant une activité du même type. De telles séquences homologues peuvent être obtenues par des expériences d'hybridation de l'ADN correspondant. Les hybridations peuvent être réalisées à partir de banques d'acides nucléiques, en utilisant comme sonde la séquence native ou un fragment de celle-ci, dans des conditions de stringence conventionnelles (Maniatis et al.), (Cf techniques générales de biologie moléculaire), ou, de préférence, dans des conditions de stringence élevées.

Dans un mode de réalisation particulièrement avantageux, les compositions de la présente invention comprennent en outre un élément de ciblage permettant d'orienter le transfert de l'acide nucléique. Cet élément de ciblage peut être un élément de ciblage extracellulaire, permettant d'orienter le transfert de l'acide nucléique vers certains types cellulaires ou certains tissus souhaités (cellules tumorales, cellules hépatiques, cellules hématopoiétiques, etc). Il peut également s'agir d'un élément de ciblage intracellulaire, permettant d'orienter le transfert de l'acide nucléique vers certains compartiments cellulaires privilégiés (mitochondries, noyau, etc).

Plus préférentiellement, l'élément de ciblage est lié, de manière covalente ou non covalente, au composé selon l'invention. L'élément de ciblage peut également être lié à l'acide nucléique. Selon un mode privilégié de l'invention, ledit composé est associé, via une partie hétérologue supplémentaire liée à une de ses extrémités. De telles parties peuvent notamment être des peptides de type fusogène pour favoriser la transfection cellulaire c'est-à-dire privilégier le passage de la composition transfectante ou de ses divers éléments à travers des membranes, aider à la sortie des endosomes ou encore pour traverser la membrane nucléaire. Il peut également s'agir d'un ligand de récepteur cellulaire présent à la surface du type cellulaire comme par exemple un sucre, la transférrine, l'insuline ou la protéine asialo-orosomucoïde. Il peut également s'agir d'un ligand de type intracellulaire comme une séquence signal de location nucléaire, nls, qui privilégie l'accumulation de l'ADN transfecté au sein du noyau.

Parmi les éléments de ciblage utilisables dans le cadre de l'invention, on peut citer les sucres, les peptides, les hormones, les vitamines, les cytokines, les oligonucléotides, les lipides ou des séquences ou fractions issues de ces éléments et permettant une liaison spécifique avec les récepteurs correspondants. Préférentiellement, il s'agit de sucres et/ou peptides tels que des anticorps ou fragments d'anticorps, des ligands de récepteurs cellulaires ou des fragments de ceux-ci, des récepteurs ou fragments de récepteurs, etc. En particulier, il peut s'agir de ligands de récepteurs de facteurs de croissance, de récepteurs de cytokines, de récepteurs de lectines cellulaires ou de récepteurs de protéines d'adhésion. On peut également citer le récepteur de la transferrine, des HDL et des LDL. L'élément de ciblage peut également être un sucre permettant de cibler des lectines tels que les récepteurs asialoglycoprotéiques, ou encore un fragment Fab d'anticorps permettant de cibler le récepteur du fragment Fc des immunoglobulines.

A titre illustratif de ce type d'association, on peut notamment mettre en oeuvre dans le cadre de la présente invention, un composé de type H₁-nls et plus préférentiellement un peptide possédant la séquence PKKKRKV-bAla-(KTPKKAKKP)₂(COOH) (SEQ ID N°9).

Avantageusement, le composé selon l'invention et plus particulièrement tout dérivé d'histone, de protamine ou de nucléoline peut être en outre polyglycosylé, sulfoné et/ou phosphorylé et/ou greffé à des sucres complexes ou à un composé lipophile comme par exemple une chaîne polycarbonée ou un dérivé du cholestérol.

La composition selon l'invention peut bien entendu comprendre plusieurs composés compactant l'acide nucléique, de nature différente. On peut ainsi associer un composé de type histone à un composé de type nucléoline.

Le composé selon l'invention est présent en quantité suffisante pour compacter l'acide nucléique selon l'invention. C'est ainsi que le rapport composé/acide nucléique (exprimé en poids) peut être compris entre 0,1 et 10 et plus préférentiellement entre 0,3 et 3.

En ce qui concerne l'agent de transfection présent dans la composition selon l'invention, il est préférentiellement choisi parmi les polymères cationiques et les lipofectants.

Selon la présente invention, le polymère cationique est de préférence un composé de formule générale I, 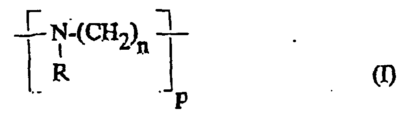 dans laquelle
- R est un atome d'hydrogène
- n est un nombre entier compris entre 2 et 10;
- p est un nombre entier, étant entendu que p est tel que le poids moléculaire moyen du polymère soit compris entre 100 et 10⁷ Da.

Il est entendu que, dans la formule (I) la valeur de n peut varier entre les différents motifs p. Ainsi, la formule (I) regroupe à la fois les homopolymères et les hétéropolymères.

Plus préférentiellement, dans la formule (I), n est compris entre 2 et 5. En particulier, les polymères de polyéthylène imine (PEI) et polypropylène imine (PPI) présentent des propriétés tout à fait avantageuses. Les polymères préférés pour la mise en oeuvre de la présente invention sont ceux dont le poids moléculaire est compris entre 10³ et 5.10⁶. A titre d'exemple, on peut citer le polyéthylène imine de poids moléculaire moyen 50 000 Da (PEI50K) ou le polyéthylène imine de poids moléculaire moyen 800 000 Da (PEI800K).

Le PEI50K ou Le PEI800K sont accessibles commercialement. Quant aux autres polymères représentés par la formule générale I, ils peuvent être préparés selon le procédé décrit dans la demande de brevet FR 94 08735.

Pour obtenir un effet optimum des compositions de l'invention, les proportions respectives du polymère et de l'acide nucléique sont de préférence déterminées de sorte que le rapport molaire R = amines du polymère / phosphates de l'acide nucléique soit compris entre 0,5 et 50, plus préférentiellement entre 5 et 30. Des résultats tout particulièrement avantageux sont obtenus en utilisant de 5 à 25 équivalents d'amines de polymère par charge d'acide nucléique.

En ce qui concerne plus particulièrement les lipofectants, on entend couvrir au sens de l'invention, sous cette dénomination, tout composé à caractère lipidique et déjà proposé à titre d'agent actif à l'égard de la transfection cellulaire d'acides nucléiques. De manière générale, il s'agit de molécules amphiphiles comprenant au moins une région lipophile associée ou non à une région hydrophile. A titre représentatif de la première famille de composés, on peut notamment proposer les lipides susceptibles de former des lipisomes comme les POPC, phosphatidylserine, phosphatidylcholine, cholestérol, maléimidophénylbutyrylphosphatidyléthanolamine, lactosylcéramide en présence ou non de polyéthylène glycol pour former des liposomes furtifs ou, avec ou sans anticorps ou ligands, pour former des immunoliposomes ou des liposomes ciblés.

Selon un mode particulier de l'invention, l'agent lipidique mis en oeuvre possède une région cationique. Cette région cationique, préférentiellement polyamine, chargée cationiquement, est capable de s'associer de manière réversible avec l'acide nucléique, chargé négativement. Cette interaction compacte fortement l'acide nucléique. La région lipophile rend cette interaction ionique inaccessible au milieu aqueux externe, en recouvrant la particule nucléolipidique formée d'une pellicule lipidique.

On sait ainsi qu'un lipide cationique chargé positivement, le chlorure de N-[1-(2,3-dioleyloxy)propyl]-N,N,N-triméthylammonium (DOTMA), interfère, sous la forme de liposomes ou de petites vésicules, spontanément avec de l'ADN, qui est chargé négativement, pour former des complexes lipides-ADN, capables de fusionner avec les membranes cellulaires, et permet ainsi la délivrance intracellulaire de l'ADN. Depuis le DOTMA, d'autres lipides cationiques sont proposés sur ce modèle de structure : groupe lipophile associé à un groupement amino via un bras dit "spacer". Parmi ceux-ci, on peut plus particulièrement citer ceux comprenant à titre de groupement lipophile deux acides gras ou un dérivé du cholestérol, et comportant, en outre, le cas échéant, à titre de groupement amino, un groupement d'ammonium quaternaire. Les DOTAP, DOBT ou le ChOTB peuvent notamment être cités à titre représentatifs de cette catégorie de lipides cationiques. D'autres composés, comme les DOSC et ChOSC, se caractérisent par la présence d'un groupement choline à la place du groupement d'ammonium quaternaire. Une autre catégorie de lipides cationiques, les lipopolyamines, a également été décrite. Dans ce type de composés, le groupement cationique est représenté par le radical L-5carboxyspermine qui contient quatre groupements d'ammonium, deux primaires et deux secondaires. Les DOGS et DPPBS en font notamment partie. Ces lipopolyamines sont tout particulièrement efficaces pour la transfection de cellules endocrines primaires.

Avantageusement, les lipofectants convenant à l'invention peuvent également être choisis parmi des lipopolyamines dont la région polyamine est représentée par la spermine ou la thermine. Quant à la région lipophile, elle est représentée par au moins une chaîne hydrocarbonée, saturée ou non, du cholestérol, un lipide naturel ou un lipide synthétique capable de former des phases lamellaires ou hexagonales, liée de manière covalente à la région hydrophile.

La demande de brevet EP 394 111 décrit d'antres lipopolyamines de formule générale III susceptibles d'être mises en oeuvre dans le cadre de la présente invention. 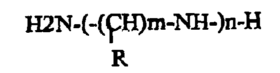 dans laquelle R représente notamment un radical de formule générale (R₁R₂) N-CO-CH-NH-CO- .

A titre représentatif de ces lipopolyamines on peut plus particulièrement citer la dioctadécylamidoglycyl spermine (DOGS) et la 5-carboxyspermylamide de la palmitoylphosphatidylethanolamine (DPPES).

Les lipopolyamines décrites dans la demande de brevet FR 94 14596 peuvent également être utilisées avantageusement à titre d'agent de transfection selon l'invention. Elles sont reptésentécs par la formule générale IV ci-dessus dans laquelle R représente 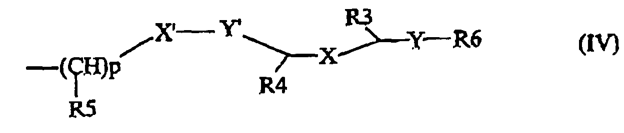 avec
- X et X représentant, indépendamment l'un de l'autre, un atome d'oxygène, un groupement méthylène -(CH₂)_{q}- avec q égal à 0, 1, 2 ou 3, ou un groupement amino
   -NH- ou -NR'- avec R' représentant un groupement alkyle en C₁ à C₄,
- Y et Y' représentant indépendamment l'un de l'autre un groupement méthylène, un groupement carbonyle ou un groupement C=S,
- R₃, R₄ et R₅ représentant indépendamment l'un de l'autre un atome d'hydrogène ou un radical alkyle, substitué ou non, en C₁ à C₄, avec p pouvant varier entre 0 et 5,
- R₆ représentant un dérivé du cholestérol ou un groupement alkyle amino
   -NR₁R₂ avec R₁ et R₂ représentant indépendamment l'un de l'autre un radical aliphatique, saturé ou non, linéaire ou ramifié en C₁₂ à C₂₂.

A titre représentatif de ces lipopolyamines on peut tout particulièrement citer le (Dioctadécyl-carbamoylméthoxy)-acétate de 2-5-bis-(3-amino-propylamino)-pentyle et le (Dioctadécyl-carbamoylméthoxy)-acétate de 1,3-bis-(3-amino-propylamino)-2 propyle dit ci-après lipopolyamine A.

Les demandes de brevet EP 394 111 et FR 94 145 96 décrivent également un procédé utilisable pour la préparation des lipopolyamines correspondantes.

Enfin plus récemment, de nouvelles lipopolyamines, valorisables également dans le cadre de la présente invention, ont été décrites dans la demande de brevet FR 95/134 90. A titre représentatif de ces lipopolyamines on peut plus particulièrement mentionner celles qui suivent:
- lipopolyamine B:

   {H₂N(CH₂)₃}₂N(CH₂)₄N{(CH₂)₃NH₂}(CH₂)₃NHCH₂COGlyN[(CH₂)₁₇-CH₃]₂ (RP120525)
- lipopolyamine C:

   H₂N(CH₂)₃NH(CH₂)₄NH(CH₂)₃NHCH₂COGlyN[(CH₂)₁₈]₂ (RP120535)
- lipopolyamine D:

   H₂N(CH₂)₃NH(CH₂)₄NH(CH₂)₃NHCH₂COArgN[(CH₂)₁₈] (RP120531)

De manière particulièrement avantageuse, on peut utiliser dans le cadre de l'invention la dioctadécylamidoglycyl spermine (DOGS) la 5-carboxyspermylamide de la palmitoylphosphatidylethanolamine (DPPES), le (Dioctadécyl-carbamoylméthoxy)-acétate de 2-5-bis-(3-amino-propylamino)-pentyle, le (Dioctadécyl-carbamoylméthoxy)-acétate de 1,3-bis-(3-amino-propylamino)-2 propyle, le {H₂N(CH₂)₃}₂N(CH₂)₄N{(CH₂)₃NH₂}(CH₂)₃NHCH₂COGlyN[(CH₂)₁₇-CH₃]₂., le H₂N(CH₂)₃NH(CH₂)₄NH(CH₂)₃NHCH₂COGlyN[(CH₂)₁₈]₂ ou le H₂N(CH₂)₃NH(CH₂)₄NH(CH₂)₃NHCH₂COArgN[(CH₂)₁₈].

Pour obtenir un effet optimum des compositions de l'invention, les proportions respectives de la polyamine et de l'acide nucléique sont de préférence déterminées de sorte que le rapport R charges positives de l'agent de transfection/charges négatives de l'acide nucléique soit compris entre 0,1 et 10 et plus préférentiellement entre 0,5 et 6.

La présence d'un composé selon l'invention au sein d'une composition transfectante permet avantageusement de diminuer considérablement la quantité en agent de trausfection. Il s'en suit une toxicité nettement amoindrie qui rend désormais possible par exemple la transfection de cellules sensibles à, l'origine à l'agent de transfection comme par exemple les cellules hématopolétiques avec les lipopolyamines. Enfin, comme le démontrent les exemples ci-après, le pouvoir transfectant des compositions selon l'invention est supérieur à celui obtenu avec les compositions transfectantes classiques.

Dans les compositions de la présente invention, l'acide nucléique peut être aussi bien un acide désoxyribonucléique qu'un acide ribonucléique. Il peut s'agir de séquences d'origine naturelle ou artificielle, et notamment d'ADN génomique, d'ADNc, d'ARNm, d'ARNt, d'ARNr, de séquences hybrides ou de séquences synthétiques ou semi-synthétiques. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent par ailleurs être incorporés dans des vecteurs, tels que des vecteurs plasmidiques.

Concernant plus particulièrement les acides désoxyribonucléiques, ils peuvent être simple ou double brin. Ces acides désoxyribonucléiques peuvent coder pour des gènes thérapeutiques, des séquences régulatrices de la transcription ou de la réplication, des séquences antisens, des régions de liaison à d'autres composants cellulaires, etc.

Au sens de l'invention, on entend par gène thérapeutique notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, VEGF, NT3, NT5, HARP/pléiotrophine, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidique acide phosphatase, ou encore des protéines de transfert de lipides comme la protéine de transfert des esters de cholestérol et la protéine de transfert des phospholipides, une protéine de liaisons des HDL ou encore un récepteur choisi par exemple parmi les récepteurs LDL, récepteurs des chylomicrons-remnants et les récepteurs scavenger, la dystrophine ou une minidystrophine (FR 9111947), la protéine GAX, la protéine CFTR associée à la mucoviscidose, les gènes suppresseurs de tumeurs : p53, Rb, RaplA, DCC, k-rev, etc (FR 93 04745), les gènes codant pour des facteurs impliqués dans la coagulation : Facteurs VII, VIII, IX, les gènes intervenant dans la réparation de l'ADN, les gènes suicides (thymidine kinase, cytosine déaminase), etc.

Le gène thérapeutique peut également être un gène ou une séquence antisens, dont l'expression dans la cellule cible permet de contrôler l'expression de gènes ou la transcription d'ARNm cellulaires. De telles séquences peuvent, par exemple, être transites dans la cellule cible en ARN complémentaires d'ARNm cellulaires et bloquer ainsi leur traduction en protéine, selon la technique décrite dans le brevet EP 140 308. Les autisens comprennent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, ou encore spécifiques de tumeurs (EP 259 212).

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc. En outre, ces séquences d'expression peuvent être modifiées par addition de séquences d'activation, de régulation, etc.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle.

Plus préférentiellement, les compositions de l'invention comprennent en outre, un ou plusieurs lipides neutres. De telles compositions sont particulièrement avantageuses, notamment lorsque le rapport R est faible. La demanderesse a en effet montré que l'addition d'un lipide neutre permet d'améliorer la formation des particules nucléolipidiques et, de manière surprenante, de favoriser la pénétration de la particule dans la cellule en déstabilisant sa membrane.

Plus préférentiellement, les lipides neutres utilisés dans le cadre de la présente invention sont des lipides à 2 chaînes grasses.

De manière particulièrement avantageuse, on utilise des lipides naturels ou synthétiques, zwitterioniques ou dépourvus de charge ionique dans les conditions physiologique. Il peuvent être choisis plus particulièrement parmi la dioleoylphosphatidyléthanolamine (DOPE), le oléoyl-palmitoylphosphatidyléthanolamine (POPE), le di-stéaroyl, -palmitoyl, -mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines)ou encore les asialogangliosides (tels que notamment les asialoGM1 et GM2).

Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Préférentiellement, les compositions de l'invention, mettant en oeuvre à titre d'agent de transfection un lipofectant, comprennent de 0,1 à 20 équivalents de lipide neutre pour 1 équivalent de lipopolyamine, et, plus préférentiellement, de 1 à 5. Dans le cas où l'agent de transfection est un polymère cationique, les compositions de l'invention comprennent, en plus du polymère cationique dans les rapports cités ci-avant, de 0,1 à 20 équivalents molaires de lipide neutre pour 1 équivalent molaire de phosphate de l'acide nucléique, et, plus préférentiellement, de 1 à 5.

Les compositions selon l'invention peuvent être formulées en vue d'administrations par voie topique, cutanée, orale, rectale, vaginale, parentérale, intranasale, intraveineuse, intratausculaire, sous-cutanée, intraoculaire, transdermique, etc... De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée.

Elles peuvent être avantageusement utilisées pour transfecter une grande variété de type cellulaire comme par exemple les cellules hematvpoïétiques, les lymphocytes, les hépatocytes, les cellules endothéliales, les cellules de mélanomes, de carcinomes et de sarcomes, les cellules musculaires lisses, les neurones et les astrocytes.

La présente invention propose également l'utilsiation de la composition pharmaceutique pour le traitement de maladies utilisant la transfection in vitro et/ou ex vivo d'un acide nucléique apte à corriger ladite maladie en association avec un agent de transfection de type polymère cationique ou lipofectant, et un composé tel que défini ci-avant. Plus particulièrement, la composition pharmaceutique selon l'invention est utilisée pour la fabrication d'un médicament destiné aux traitements de maladies résultant d'une déficience en un produit protéique ou nucléique et l'acide nucléique administré code pour ledit produit protéique ou contient la séquence correspondant audit produit nucléique. Les compositions selon l'invention sont particulièrement intéressantes pour leur biodisponibilité et leur haut niveau de transfection.

La présente invention concerne également toute utilisation d'un composé constitué en tout ou partie, de motifs peptidiques (KTPKKAKKP) et/ou (ATPAKKAA) avec le nombre de ces motifs pouvant varier entre 2 et 10, pour, lorsqu'ils sont couplés à un ligand de récepteur cellulaire, un anticorps ou dérivé d'anticorps, cibler un acide nucléique vers des cellules exprimant les récepteurs ou anti-gènes correspondants. Dans cette perspective, un ligand, anticorps ou dérivé d'anticorps potentiel est couplé audit composé et l'on apprécie le pouvoir de transfection de cette molécule chimère comparativement au composé seul.

La présente invention couvre également toute utilisation d'un oligopeptide sélectionné parmi:
(ATPAKKAA)₂(COOH),
(KTPKKAKKP)₂(COOH),
ATPKKSAKKTPKKAKKP(COOH),
KKAKSPKKAKAAKPKKAPKSPAKAKA(COOH), SRSRYYRQRQRSRRRRRR (COOH) et RRRLHRIHRRQHRSCRRRKRR(COOH).
pour effectuer le transfert in vitro, et/ou ex vivo d'au moins un acide nucléique, ledit oligonucléotide étant associé ou non à un élément de ciblage.

La présente invention sera plus complètement décrite à l'aide des exemples qui suivent, qui doivent être considérés comme illustratifs et non limitatifs.

### Matériel et Méthodes :

### 1. Plasmides utilisés pour le transfert de gènes in vivo

Les constructions utilisées pour mettre en évidence l'activité des compositions de l'invention sont des plasmides comportant le gène codant pour la luciférase (Luc)

Il s'agit notamment de plasmides pCMV luc, pXL 2621, pXL 2622, qui tous contiennent le gène codant pour la luciférase (tiré du vecteur pGL2, Promega) en aval du promoteur du cytomegalovirus (CMV) extrait de pCDNA3 (Invitrogen). pCMV luc et pXL2622 dérivent d'un vecteur pGL2, pXL 2621 d'un vecteur pGL2 contrôle, et dans tous ces vecteurs le promoteur SV40 a été remplacé par le promoteur CMV.

De manière générale les plasmides sont obtenus par le technique de précipitation au PEG (Ausubel), et stockés dans du Tris 10mM EDTA 1mM pH 8 à 4 °C à une concentration d'enviroxt 10 µg d'ADN par µl.

### 2. Composés utilisés selon l'invention:

### H: KTPKKAKKPKTPKKAKKP(COOH) 18 AA

### 2.1 N: ATPAKKAAATPAKKAA(COOH)

Cet oligopeptide a été synthétisé sous forme de sel d'acide trifluoroacétique au moyen d'un synthétiseur de peptides Applied Biosystem 431A, sur une résine HMP (Applied Biosystem) et selon une stratégie FMOC. Après la synthèse, le peptide a été libéré de la résine par traitement 90 minutes en présence d'une solution eau/TFA 1/19. Après filtration, la solution est concentrée sur évaporateur rotatif, puis le peptide est précipité 2 fois par addition d'éther tertiobutylméthylique à partir de solution dans le TFA. Le culot final est lavé par l'éther tertiobutylméthylique puis séché. Le peptide est solubilisé dans 5 ml d'eau, filtré et purifié par HPLC phase inverse sur colonne C18 100 A (Biorad RSL). le peptide est purifié par à l'aide d'un gradient de 0 à 25%d'acétonitrile, 0,07% TFA dans l'eau 0,07% TFA. La pureté du peptide obtenu est supérieure à 95% et sa solubilité dans l'eau de 100 mg/ml.

### 2.2 nls: PKKKRKV

Cet oligopeptide a été synthétisé sous forme de sel d'acide trifluoroacétique selon le protocole décrit ci-dessus en utilisant pour le clivage une solution eau/TFA/phénol/éthanedithiol/thioanisole 2/ 40/3/1/2 (v/v). La pureté du peptide obtenu est supérieure à 95% et sa solubilité dans l'eau de 100 mg/ml à pH 2,1.

### 2.3 H: KTPKKAKKPKTKKAKKP(COOH) et nls-H: PKKKRKV-bAla-KTPKKAKKPKTKKAKKP(COOH)

Ces oligopeptides sont synthétisés sous forme de sel d'acide trifluoroacétique selon le protocole décrit ci-dessus. Pour ce faire, on divise la résine en deux lots. Un premier lot destiné à la synthèse de H est traité pour le clivage avec une solution TFA/phénol/éthanedithiol/thioanisole/eau 40/3/1/2/2 (v/v). La pureté du peptide obtenu est supérieure à 90% et sa solubilité dans l'eau de 10 mg/ml à pH 2,1. Sur le second lot de résine la synthèse est poursuivie de manière à obtenir nls-bAla-H. La solution de clivage mise en oeuvre est identique à la précédente. La pureté du peptide obtenu est supérieure à 95 % et sa solubilité dans l'eau de 10 mg/ml à pH 2,1.

### 2.4 PR1 SRSRYYRQRQRSRRRRRR et

### PR2: RRRLHRIHRRQHRSCRRRKRR

Ces oligopeptides sont assemblés en plusieurs étapes, en synthèse phase solide selon la technique BocBenzyle. La résine de départ est une résine Boc-L-Arg(Tos)Pam ( 0,48meq/g).. Le procédé de déprotection et couplage mis en oeuvre est le suivant:

| | |
|---|---|
| 1- 55 % TFA dans du dichlorométhane (DCM) | 1 x 2 mn |
| 2- 55 % TFA dans du dichlorométhane | 1 x 30 mn |
| 3- DCM | 2 x 1mn |
| 4- DMF | 3 x 1 mn |
| 5- Couplage | |
| 6- DMF | 2 x 1mn |
| 7- DCM | 2 x1 mn |

Pour chaque étape, on utilise 10ml de solvant par gramme de résine peptide mis en oeuvre. Le couplage de tous les acides aminés (en triple excès) est effectué dans le DMF en présence de BOP, Hobt et DIEA. Chaque étape de couplage est contrôlée par le test ninhydride

Le peptide final est récupéré de la résine et entièrement déprotégé avec de l'acide fluorhydrique liquide. On utilise 10 ml de HF par gramme de peptide résine à 0°C pendant 45 minutes en présence de para-crésol et d'éthanedithiol. Après évaporation de l'acide fluorhydrique le mélange réactionnel brut est lavé avecde l'éther, dissous dans de l'acide trifluoroacétique, précipité à l'éther et séché.

### 3. Agents lipofectants mis en oeuvre

Lipopolyamine A:
   (Dioctadécyl-carbamoylméthoxy)-acétate d1,3-bis-(3-amino-propylamino)-2 propyle (RP115335)
Lipopolyamine B:

   {H₂N(CH₂)₃}₂N(CH₂)₄N{(CH₂)₃NH₂}(CH₂)₃NHCH₂COGlyN[(CH₂)₁₇-CH₃]₂ (RP120525)
Lipopolyamine C:

   H₂N(CH₂)₃NH(CH₂)₄NH(CH₂)₃NHCH₂COGlyN[(CH₂)₁₈]₂ (RP120535)
Lipopolyamine D:

   H₂N(CH₂)₃NH(CH₂)₄NH(CH₂)₃NHCH₂COArgN[(CH₂)₁₈]₂ (RP120531)

### EXEMPLE 1 :

### Transfert d'acide nucléique in vitro dans des cellules NIH 3T3

Cet exemple décrit le transfert d'acides nucléiques in vitro (sur cultures cellulaires) au moyen d'une composition selon l'invention comprenant l'acide nucléique, un composé selon l'invention, et une lipopolyamine dans différentes conditions de pH et de tampon.
1. On prépare un mélange de 10µl composé comme suit:
- 0,5µg d'ADN plasmidique pCMV-luc, 0,25µg d'un composé selon l'invention, dans une solution tampon telle qu'identifiée,
- du dioctadécylamidoglycyl spermine (DOGS) 40mM dans des rapports de charges X tels qu'indiqués dans chacun des essais.

2- 5.10⁴ cellules des lignées NIH3T3 sont incubées avec le mélange précédent à 37°C, sous une atmosphère de CO₂ à 5 % pendant 4 heures. Les cellules sont ensuite lavées et remises en culture pendant 48 heures dans un milieu contenant 10 % de sérum (DMEM 10 %SVF).
Le tapis cellulaire est ensuite lysé dans 50µl de tampon lyse (Promega), récupéré puis centrifugé à 20 000g pendant 5 minutes.
L'activité luciférase est mesurée sur 4µl du surnageant en ajoutant 20µl de substrat (Promega). La lecture se fait sur luminomètre LKB en cumulant les RLU (relative lights units) sur 20 secondes.
Les résultats sont présentés dans les tableaux 1 et 2 ci-après.
3. Compaction dans NaCl 150mM, HEPES* (* N-(2-hydroxyéthyl)pipérazine-N'-(acide2-éthyl-sulfonique) 10mM à pH 7,1

**TABLEAU 1**

| **Rapport de charge DOGS/ADN** | **sans composé (R.L.U)** | **avec H (R.L.U)** |
|---|---|---|
| 0,8 X | 32 | 850 |
| 1,8 X | 220 | 23700 |
| 3 X | 5400 | 21750 |

4. Compaction dans D-glucose 5 %. NaCl 150mM, avec HEPES 10mM (pH de la solution de compaction: 7,2)

**TABLEAU 2**

| **Rapport de charge DOGS/ADN** | **sans composé (R.L.U)** | **avec H (R.L.U)** | **avec N (R.L.U)** |
|---|---|---|---|
| 0,8 X | 880 | 44000 | 1450 |
| 1,8 X | 6600 | 156500 | - |
| 3 X | 22000 | 297500 | 90300 |

Dans l'ensemble des essais, on note des résultats supérieurs lorsque la composition compactante associe à DOGS un composé selon l'invention. Il s'avère possible de diminuer de manière importante la quantité en DOGS sans porter préjudice à la capacité de transfection de la composition correspondante.

### EXEMPLE 2 :

### Essais de transfection en présence d'un lipide neutre

On prépare un mélange de 10µl composé comme suit:
- 0,5µg d'ADN plasmidique pCMV-luc, 0,25µg d'un composé selon l'invention, dans une solution tampon NaCl 150mM, tampon phosphate 10mM pH 7,4,
- 40mM de dioctadécylamidoglycyl spermine (DOGS) dans des rapports de charges X tels qu'indiqués dans chacun des essais en présence de dioléoylphosphatidyléthanolamine (DOPE) avec DOGS/DOPE égal à 1/2.

Dans ce cas particulier, une solution éthanolique de DOGS à 40 mM est mélangée avec un volume égal d'une solution de dioléoylphosphatidyléthanolamine (DOPE) à 80 mM, préparée dans un mélange chloroforme/éthanol (1/5). Ainsi pour un équivalent de DOGS la composition contient deux équivalents de DOPE.

10⁵ cellules des lignées NIH3T3 sont incubées avec ce mélange dans les conditions décrites dans l'exemple précédent. A l'issue de l'incubation, ces cellules sont traitées selon le protocole de ce même exemple. Les résultats sont présentés dans le tableau ci-après.

**TABLEAU 3**

| **Rapport de charge DOGS/ADN** | **sans composé (R.L.U)** | **avec H (R.L.U)** | **avec chimère nls-H (R.L.U)** |
|---|---|---|---|
| 0,8 X D/D | 24 | 3175 | 760 |
| 1 X D/D | 19 | 7410 | 2380 |
| 1,8 X D/D | 1800 | 39500 | 52800 |

Ces résultats confirment ceux observés en exemple 1. En présence d'un composé basique selon l'invention et plus particulièrement de H, il est possible de réduire de moitié la quantité en lipofectant .

### EXEMPLE 3 :

### Variation du rapport composé selon l'invention/ADN au sein d'une composition transfectante selon l'invention.

### 3.1: En présence d'une composition DOGS

1. On prépare un mélange de 10µl composé de:
- 0,75µg d'ADN plasmidique pCMV-luc, et d'un composé selon l'invention dans le rapport indiqué, dans une solution tampon D-glucose 5 %, NaCl 150mM, avec HEPES 10mM (pH de la solution de compaction : 7,2),
- du dioctadécylamidoglycyl spermine (DOGS) 40mM dans un rapport de charge 1,8X.

2.- 4.10⁵ cellules des lignées NIH3T3 sont incubées avec le mélange précédent à 37°C, sous une atmosphère de CO₂ à 5 % pendant 4 heures. Les cellules sont ensuite lavées et remises en culture pendant 48 heures dans un milieu contenant 10 % de sérum (DMEM 10 %SVF). Le tapis cellulaire est ensuite lysé, 45 heures après la transfection, dans 100 µl de tampon lyse (Promega), récupéré puis centrifugé à 20000g pendant 5 minutes. L'activité luciférase est mesurée sur 5 µl du surnageant en ajoutant 25 µl de substrat (Promega). La lecture se fait sur luminomètre LKB en cumulant les RLU (relative lights units) sur 20 secondes.
Les résultats figurent dans le tableau 4 ci-après.

**TABLEAU 4**

| **PEP/ADN w/v** | **avec H (R.L.U)** | **avec H-nls (R.L.U)** | **avec N (R.L.U)** |
|---|---|---|---|
| 0 | 2 150 | 2 150 | 2 150 |
| 0,25 | 3 300 | 38000 | 35 000 |
| 0,5 | 45 000 | 100 000 | 35 000 |
| 1 | 84 000 | 105 000 | 13 000 |
| 2 | 105 000 | 200 000 | 20 000 |

### 3.2: En présence de DOGS/DOPE 1,8X

On procède selon le protocole décrit précédemment en 3.1 mais en utilisant à titre d'agent de transfection une solution de 40mM de dioctadécylamidoglycyl spermine (DOGS) 1,8X en présence de dioléoylphosphatidyléthanolamine (DOPE) avec DOGS/DOPE égal à 1/2, préparée selon le mode opératoire présenté en exemple 2.

Le tableau 5 rend compte des résultats observés.

**TABLEAU 5**

| **COMPOSE /ADN w/v** | **H (R.L.U)** | **H-nls (R.L.U)** | **N (R.L.U)** |
|---|---|---|---|
| 0 | 580 | 580 | 580 |
| 0,25 | 13 000 | 5600 | 7600 |
| 0,5 | 11 000 | 18500 | 1 800 |
| 1 | 14100 | 36000 | 13 600 |
| 2 | 16 500 | 58 000 | 14 100 |

### EXEMPLE 4 :

### Variation du rapport composé selon l'invention/ADN au sein d'une composition transfectante selon l'invention en utilisant un agent de transfection autre que le DOGS.

### 3.1: En présence du (Dioctadécyl-carbamoylméthoxy)-acétate de 1,3-bis-(3-amino-propylamino)-2 propyle(lipopolyamine A)

1. On prépare un mélange de 10µl composé de:
- 0,55µg d'ADN plasmidique pCMV-luc, et d'un composé selon l'invention dans le rapport indiqué, dans une solution tampon NaCl 150mM,
- du (Dioctadécyl-carbamoylméthoxy)-acétate de 1,3-bis-(3-amino-propylamino)-2 propyle dans dans les rapport de charge indiqués.

2.- 5.10⁴ cellules des lignées NIH3T3 sont incubées avec le mélange précédent à 37°C, sous une atmosphère de CO₂ à 5 % pendant 4 heures. Les cellules sont ensuite lavées et remises en culture pendant 48 heures dans un milieu contenant 10 % de sérum (DMEM 10 %SVF). Le tapis cellulaire est ensuite lysé, 45 heures après la transfection, dans 100 µl de tampon lyse (Promega), récupéré puis centrifugé à 20000 g pendant 5 minutes. L'activité luciférase est mesurée sur 10 µl du surnageant en ajoutant 50 µl de substrat (Promega). La lecture se fait sur luminomètre Berthold lumat 9501® en cumulant les RLU (relative lights units) sur 10 secondes. Les résultats figurent dans le tableau 6 ci-après.

**TABLEAU 6**

| **LIPOFECTANT** | **sans composé .10**^{**6**}**.R.L.U** | **avec H .10**^{**6**}**.R.L.U** | | | **avec nls-H .10**^{**6**}**.R.L.U** | | |
|---|---|---|---|---|---|---|---|
| **Rapport NH**_{**2**} **du lipofectant / phosphates de l'ADN** | **Rapport composé/ADN** | | | | | | |
| | **0** | **0,5** | **1** | **2** | **0,5** | **1** | **2** |
| 1,8X | 3,9 | 30,4 | 32,7 | 4,1 | 52,4 | 58,5 | 39, 3 |
| 3X | 8,9 | 41,5 | 61,3 | 16,6 | 64,1 | 60,4 | 49, 2 |
| 6X | 6,2 | 23,6 | 22,3 | 15,6 | | | |

### 4.2: En présence du PEI 800K

1. On procède selon le protocole décrit en 4.1 et dans un même milieu, en utilisant à titre de lipofectant le PEI 800K. Les résultats figurent dans le tableau 7 ci-après.

**TABLEAU 7**

| **PEI 800K** | **Sans composé .10**^{**6**}**.R.L.U** | **H .10**^{**6**}**.R.L.U** | | | **nls-H .10**^{**6**}**.R.L.U** | | |
|---|---|---|---|---|---|---|---|
| Equivalent d'amines de polymère par phosphate de l'ADN | Rapport composé/ADN | | | | | | |
| | **0** | **0,5** | **1** | **2** | **0,5** | **1** | **2** |
| 6 | 7,7 | 4,9 | 7 | 12,3 | 4 | 7,3 | 8,8 |
| 9 | 5 | 8 | 11,6 | 4 | 16,3 | 11 | 12,1 |
| 12 | 7,5 | 11,3 | 17,1 | 1,2 | | | |

### EXEMPLE 5

### Transfert in vivo dans des cellules de muscles.

Les essais correspondants sont réalisés en mettant en oeuvre les matériels et protocoles suivants:
Modèle: L'injection se pratique dans le muscle tibial cranial de souris C57 b16 ou OF1 adultes (âgées de plus de 8 semaines)
Protocole: L'ADN est dilué à 0,5 mg/ml dans une solution qui contiendra en final NaCl 150mM, D-Glucose 5 %. Dans certains groupes, avant injection, du peptide en solution à 1 mg/ml dans l'eau est ajouté à l'ADN en quantité suffisante pour atteindre les rapports poids/poids indiqués. Une incubation d'au moins 20 minutes à température ambiante est réalisée avant de procéder à l'injection.
Détermination des résultats: Deux jours après l'injection, le muscle est prélevé, haché dans 750 µl de tampon de lyse (Promega E153A) additionné d'aprotinine (Sigma). Le prélèvement est homogénéisé dans un broyeur (Heidolf), et 10 µl servent à la mesure de l'activité luciférase. Cette mesure est éffectuée avec un luminomètre Lumat 9501 (Berthold), en totalisant l'émission émise pendant 10 secondes après addition de 50 µl de substrat luciférase (Promega) aux 10 µl de l'échantillon. Le bruit de fond mesuré avant addition de substrat est soustrait à ce total, et l'activité est exprimé en RLU (relative light units) totaux (rapportés aux 750 ml de tampon de lyse). Pour ce faire, 40 µl (20 µg d'ADN) sont injectés, en présence d'HEPES à pH 7,4 5 mM final dans la solution, puis ajout de la lipopolyamine C(RPR 120 535) dans un rapport 0,01 nmol/µl d'ADN 20 minutes avant injection:

**TABLEAU 8**

| **Peptide, Rapport peptide / ADN poids/poids** | **Moyenne RLU** | **écart type RLU** | **nombre d'animaux** |
|---|---|---|---|
| sans peptide | 15 228 125 | 14 618 681 | 6 |
| nls-H, 0,025 poids/poids +lipopolyamine C | 42 722 500 | 66 485 348 | 6 |

Ces résultats confirment l'effet bénéfique de l'association d'une lipopolyamine à un agent compactant selon l'invention sur la transfection in vivo dans le muscle d'un acide nucléique.

### EXEMPLE 6

### Transfert in vivo de compositions revendiquées dans des cellules tumorales

Les essais correspondants sont effectuées sur des souris C57/BL 6 adultes (>8 semaines) femelles portant des tumeurs de type 3LL (Lewis Lung carcinoma) obtenues par passage de fragments de tumeur d'animal à animal, implantés sous la peau au niveau du flanc.

En ce qui concerne les solutions injectées elles sont préparées comme suit: L'ADN est d'abord solubilisé dans le tampon, le peptide est alors éventuellement ajouté, et après 20 minutes une solution de lipides cationiques à forte concentration (20 ou 40 mM) est ajoutée au mélange. Après addition de tous les produits, le mélange contient, outre l'ADN, le peptide et le lipide cationique, NaCl 150mM, D-Glucose 5 % et MES 5mM pH 6,2. Dans le cas des deux dernières séries avec la lipopolyamine C (RPR 120 535), le véhicule d'injection est NaCl 75 mM et NaCl 150mM, D-glucose 5 %, respectivement.
L'injection est réalisée 20 à 30 minutes après la préparation de la solution.

On réalise l'injection de chaque composition transfectante (voir tableaux 9 et 10 pour leurs spécificités respectives) dans la tumeur 7 jours après implantation, la souris étant anesthésiée avec un mélange Kétamine 130 mg/kg+ Xylazine (4 mg/mg).

Deux jours après l'injection, on prélève le tissus tumoral qui est pesé puis haché et broyé dans 500 µl tampon de lyse (Promega Cell Lysis Buffer E153 A). Après centrifugation (20000 g pendant 10 minutes), on prélève 10 µl qui servent à l'évaluation de l'activité luciférase par mesure de l'émission lumineuse totale obtenue après mélange avec 50 µl de réactif (Promega Luciferase Assay Substrate) dans un luminomêtre Lumat LB 9501 (Berthold), intégration sur 10 secondes.

L'activité résultante est exprimée en RLU (Relative Lights Units) estimés dans la totalité du surnageant de lyse tumorale, ou en RLU par µg d'ADN injecté.

Le tableau 9 rend compte de résultats obtenus en présence de diverses lipopolyamines A, B, C ou D et le tableau 10 en présence de PEI.

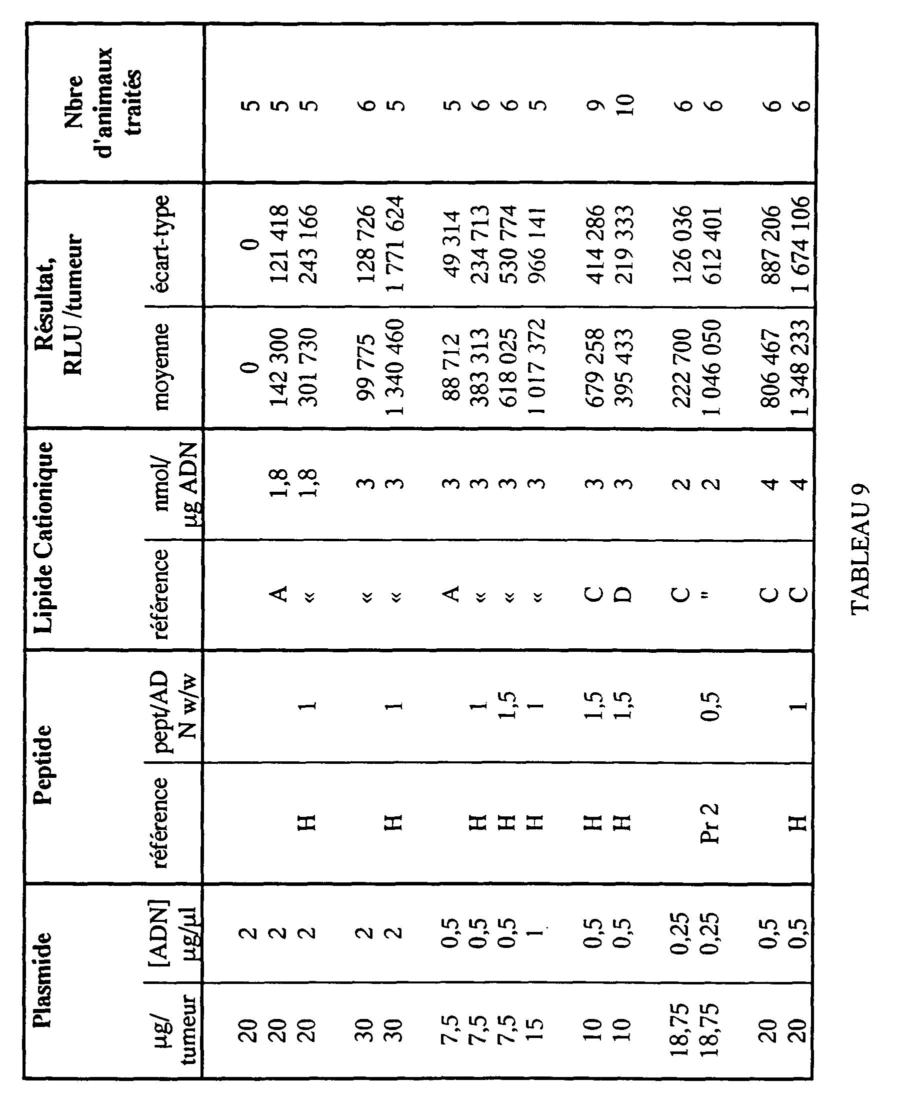

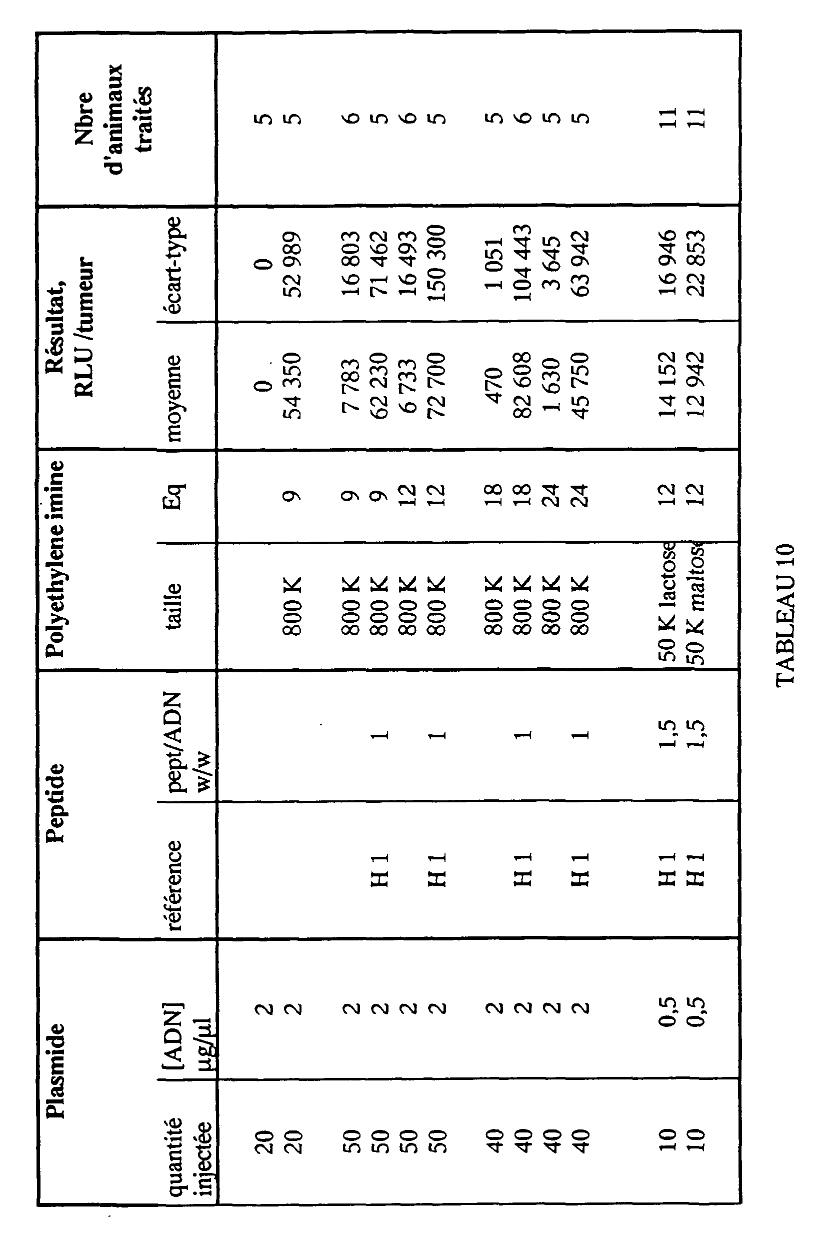

### EXEMPLE 7

### Transfert d'acide nucléique in vitro dans des cellules 3LL

Cet exemple décrit le transfert d'acides nucléiques in vitro (sur cultures cellulaires) au moyen d'une composition selon l'invention comprenant l'acide nucléique, un composé selon l'invention choisi parmi les dérivés de protamines et une lipopolyamine dans une solution de NaCl 75 mM final.

On prépare un mélange de 10 µl composé comme suit:
- 0.5 µg d'ADN plasmidique pCMV-luc, 0,5 µg de PR1 ou PR2 un composé selon l'invention, dans une solution de Nacl 75 mM final
- Lipopolyamine C (RPR 120535) dans des rapport de charges tels qu'indiqués dans chacun des essais répertoriés dans le tableau 11 ci-après.
1.10⁵ cellules 3LL (dans 250 µl de milieu de culture DMEM avec 10 % de sérum de veau foetal) sont incubées avec le mélange précédent à 37°C sous une atmosphère de CO₂ à 5 % pendant 4 heures. 500 µl de milieu de culture sont ajoutés et les cellules sont remises en culture. Le lendemain, les cellules sont lavées et remises en culture pendant 24 heures dans le mëme milieu contenant 10 % de sérum de veau foetal. Le tapis cellulaire est ensuite lysé dans 100 µl de tampon de lyse (Promega). L'activité luciférase est mesurée en ajoutant 50 µl de substrat (Promega). La lecture se fait sur le luminomètre LB en cumulant les RLU (relative Light Unit) sur 10 secondes.

Les tableaux 11 et 12 rendent compte respectivement des essais avec PR1 et PR2.

**TABLEAU 11**

| **RAPPORT DE CHARGE Lipopolyamine C/ADN** | **SANS COMPOSE (RLU)** | **AVEC PR2 (RLU)** |
|---|---|---|
| 4X | 107289 | 447214 |
| 6X | 77 396 | 1 182 641 |
| 8X | 14 512 | 729 285 |

**TABLEAU 12**

| **RAPPORT DE CHARGE Lipopolyamine C/ADN** | **SANS COMPOSE (RLU)** | **AVEC PR2 (RLU)** |
|---|---|---|
| 4X | 12 037 | 6 304 |
| 6X | 901 | 113 113 |
| 8X | 328 | 294 743 |

### EXEMPLE 8:

### Injections in vivo de peptides compactants selon l'invention sans lipofectant dans des tumeurs

Modèle:
- souris de type Swiss/nude femelles adulte
- tumeurs expérimentales induites après injection de 10⁷ cellules 3T3 HER2 par voie sous-cutanée au niveau du flanc.
- injection du mélange de transfection 7 à 12 jours après l'injection des cellules. La solution d'ADN compacté ou non à du peptide est injectée directement dans la tumeur avec une seringue de type Hamilton.
- deux jours après l'injection, prélèvement du tissus tumoral, qui est pesé puis haché et homogénéisé dans 750 µl tampon de lyse (Promega Cell Lysis Buffer E153 A). Après centrifugation (20 000 g pendant 10 minutes), on prélève 10 µl qui servent à l'évaluation de l'activité luciférase par mesure de l'émission lumineuse totale obtenue après mélange avec 50 µl de réactif (Promega Luciferase Assay Substrate) dans un luminomêtre Lumat LB 9501 (Berthold), intégration sur 10 secondes.

L'activité résultante est exprimée en RLU (Relative Lights Units) estimés dans la totalité du surnageant de lyse tumorale.

Protocole: L'ADN est dilué à 0,5 mg/ml dans une solution qui contiendra en final les sels, le tampon et le glucose en quantité finale telle que mentionnée dans le tableau de résultats. Dans certains groupes, avant injection, du peptide en solution à 1 mg/ml dans l'eau est ajouté à l'ADN en quantité suffisante pour atteindre les rapports poids/poids indiqués. Une incubation d'au moins 20 minutes à température ambiante est réalisée. Les souris recoivent une injection de 20 µl, soit 10 µg d'ADN au total par tumeur.

**TABLEAU 13**

| **Tampon** | **Peptide** | | **Résultat, RLU /tumeur** | | **Nbre d'animaux traités** |
|---|---|---|---|---|---|
| NaCl/gluc/ MES ou HEPES 5mM | reférence | pept/ ADN w/w | moyenne | écart-type | |
| 150/HEPES | | | 1 689 938 | 1 388 072 | 6 |
| | nls-H | 0,02 | 6819100 | 5 860 709 | 6 |
| 150/5/HEPES | | | 369 563 | 577901 | 6* |
| | nls-H1 | 0,1 | 1 654 313 | 2 145 147 | 6* |
| | nls-H1 | 0,02 | 4 031 000 | 1 007 896 | 6* |
| | | | 931 275 | 214 229 | 4 |
| | H | 0,1 | 3 420 432 | 1285704 | 6 |

| | | | | | |
|---|---|---|---|---|---|
| *: souris endormies pendant l'injection, par narco-neuroleptanalgésie avec un mélange Imalgène+ Rompun (Ketamine 130 mg/kg, Xylasine 4 mg/kg, voie intra-péritonéale). | | | | | |

Ces résultats montrent que, dans des tumeurs susceptibles de pouvoir être transfectées par de l'ADN nu, l'addition de peptide avec des faibles rapport peptide/ADN, sans association de lipides cationiques, permet d'augmenter l'expression du gène exogène par rapport à l'ADN nu seul.

## Revendications

1. Composition pharmaceutique utile pour la transfection d'un acide nucléique contenant outre ledit acide nucléique, au moins un agent de transfection et un composé intervenant au niveau de la condensation dudit acide nucléique **caractérisée en ce que** ledit composé est constitué en tout ou partie :
- de 2 à 10 motifs peptidiques (KTPKKAKKP) et/ou (ATPAKKAA) répétés de manière continue ou non,
- ou d'un oligopeptide choisi parmi
SRSRYYRQRQRSRRRRRR(COOH) et
RRRLHRIHRRQHRSCRRRKRR(COOH).

2. Composition pharmaceutique selon la revendication 1 **caractérisée en ce que** les motifs peptidiques sont séparés entre eux par un ou plusieurs acides aminés.

3. Composition pharmaceutique selon la revendication 1 ou 2 **caractérisée en ce que** le composé intervenant au niveau de la condensation dudit acide nucléique est un oligopeptide choisi parmi (KTPKKAKKP)₂(COOH) et (ATPAKKAA)₂(COOH).

4. Composition pharmaceutique selon l'une des revendications précédentes **caractérisée en ce que** ledit composé possède une structure en feuillet β.

5. Composition pharmaceutique selon l'une des revendications 1 à 4 **caractérisée en ce que** ledit composé est en outre associé à un ligand de récepteur cellulaire.

6. Composition pharmaceutique selon la revendication 5 **caractérisée en ce que** le dit composé est le peptide PKKKRKV-bAla-(KTPKKAKKP)₂(COOH).

7. Composition pharmaceutique selon l'une des revendications 1 à 6 **caractérisée en ce que** ledit composé est en outre associé à un peptide de type fusogène favorisant la transfection cellulaire de ladite composition.

8. Composition pharmaceutique selon l'une des revendications 1 à 7 **caractérisée en ce que** ledit composé est en outre polyglycosylé, sulfoné, phosphorylé et/ou greffé à des sucres complexes ou à un agent lipophile.

9. Composition pharmaceutique selon l'une des revendications précédentes **caractérisée en ce que** l'agent de transfection est un polymère cationique ou un lipofectant.

10. Composition pharmaceutique selon la revendication 9 **caractérisée en ce que** le lipofectant est un lipide susceptible de former des lipisomes, des liposomes furtifs, des immunoliposomes ou des liposomes ciblés.

11. Composition pharmaceutique selon la revendication 9 **caractérisée en ce que** le polymère cationique est de préférence un composé de formule générale (I) : 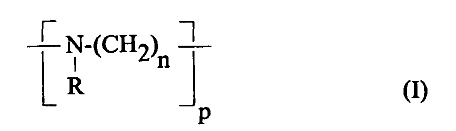 dans laquelle
- R est un atome d'hydrogène
- n est un nombre entier compris entre 2 et 10;
- p est un nombre entier,
avec p étant tel que le poids moléculaire moyen du polymère soit compris entre 100 et 10⁷ Da.

12. Composition pharmaceutique selon la revendication 9 **caractérisée en ce que** le polymère cationique est choisi parmi le polyéthylène imine (PEI) et le polypropylène imine (PPI).

13. Composition pharmaceutique selon la revendication 12 **caractérisée en ce que** le polymère est choisi parmi le polyéthylène imine de poids moléculaire moyen 50 000 Da (PEI50K) et le polyéthylène imine de poids moléculaire moyen 800 000 (PEI800K).

14. Composition pharmaceutique selon la revendication 9 **caractérisée en ce que** le lipofectant comprend au moins une région polyamine est représentée par la spermine, ou la thermine.

15. Composition pharmaceutique selon la revendication 9 **caractérisée en ce que** le lipofectant comprend au moins une région lipophile représentée par la formule générale (IV) 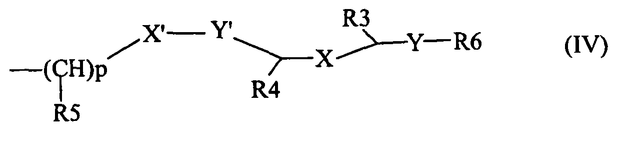 dans laquelle
- X et X' représentent, indépendamment l'un de l'autre, un atome d'oxygène, un groupement méthylène -(CH₂)_{q}- avec q égal à 0, 1, 2 ou 3, ou un groupement amino
-NH- ou -NR'- avec R' représentant un groupement alkyle en C₁ à C₄,
- Y et Y' représentent indépendamment l'un de l'autre un groupement méthylène, un groupement carbonyle ou un groupement C=S,
- R₃, R₄ et R₅ représentent indépendamment l'un de l'autre un atome d'hydrogène ou un radical alkyle, substitué ou non, en C₁ à C₄, avec p pouvant varier entre 0 et 5,
- R₆ représente un dérivé du cholestérol ou un groupement alkyle amino -NR₁R₂ avec R₁ et R₂ représentant indépendamment l'un de l'autre un radical aliphatique, saturé ou non, linéaire ou ramifié en C₁₂ à C₂₂.

16. Composition pharmaceutique selon la revendication 9 **caractérisée en ce que** le lipofectant est une lipopolyamine choisie parmi la dioctadécylamidoglycyl spermine (DOGS) la 5-carboxyspermylamide de la palmitoylphosphatidylethanolamine (DPPES), le (Dioctadécyl-carbamoylméthoxy)-acétate de 2-5-bis-(3-amino-propylamino)-pentyle ou le (Dioctadécylcarbamoylméthoxy)-acétate de 1,3-bis-(3-amino-propylamino)-2 propyle, le {H₂N(CH₂)₃}₂N(CH₂)₄N{(CH₂)₃NH₂}(CH₂)₃NHCH₂COGlyN[(CH₂)₁₇-CH₃]₂, le H₂N(CH₂)₃NH(CH₂)₄NH(CH₂)₃NHCH₂COGlyN[(CH₂)₁₈]₂ ou le H₂N(CH₂)₃NH(CH₂)₄NH(CH₂)₃NHCH₂COArgN[(CH₂)₁₈].

17. Composition pharmaceutique selon l'une quelconque des revendications 1 à 9 et 14 à 16 **caractérisée en ce que** l'agent de transfection est la dioctadécylamidoglycyl spermine (DOGS).

18. Composition pharmaceutique selon l'une des revendications précédentes **caractérisée en ce que** l'acide nucléique est un acide désoxyribonucléique.

19. Composition pharmaceutique selon l'une des revendications 1 à 17 **caractérisée en ce que** l'acide nucléique est un acide ribonucléique.

20. Composition pharmaceutique selon la revendication 18 ou 19 **caractérisée en ce que** l'acide nucléique est modifié chimiquement.

21. Composition pharmaceutique selon la revendication 18, 19 ou 20 **caractérisée en ce que** l'acide nucléique est un antisens.

22. Composition pharmaceutique selon l'une des revendications 18 à 20 **caractérisée en ce que** l'acide nucléique comporte un gène thérapeutique.

23. Composition pharmaceutique selon l'une des revendications précédentes comprenant en outre un ou plusieurs lipides neutres.

24. Composition pharmaceutique selon la revendication 23 **caractérisée en ce que** le ou les lipides neutres sont choisis parmi les lipides synthétiques ou naturels, zwitterioniques ou dépourvus de charge ionique dans les conditions physiologiques.

25. Composition pharmaceutique selon la revendication 23 ou 24 **caractérisée en ce que** le ou les lipides neutres sont choisis parmi la dioléoylphosphatidyléthanolamine (DOPE), l'oléoyl-palmitoylphos-phatidyléthanolamine (POPE), le distéaroyl, -palmitoyl, -mirystoyl phosphatidyléthanolamine ainsi que leurs dérivé N-méthylés 1 à 3 fois; les phosphatidylglycérols, les diacylglycérols, les glycosyldiacylglycérols, les cérébrosides (tels que notamment les galactocérébrosides), les sphingolipides (tels que notamment les sphingomyélines) et les asialogangliosides (tels que notamment les asialoGMl et GM2).

26. Utilisation d'une composition pharmaceutique selon l'une des revendications 1 à 25 pour le transfert in vitro, et/ou ex vivo d'acides nucléiques.

27. Utilisation d'un composé constitué en tout ou partie, de motifs peptidiques (KTPKKAKKP) et/ou (ATPAKKAA) avec le nombre de ces motifs pouvant varier entre 2 et 10, pour, lorsqu'il est couplé à un ligand de récepteur cellulaire, un anticorps ou dérivé d'anticorps, cibler, in vitro, et/ou ex vivo un acide nucléique vers des cellules exprimant les récepteurs ou anti-gènes correspondants.

28. Utilisation d'un oligopeptide sélectionné parmi :
(ATPAKKAA)₂(COOH),
(KTPKKAKKP)₂(COOH),
ATPKKSAKKTPKKAKKP(COOH),
KKAKSPKKAKAAKPKKAPKSPAKAKA(COOH),
SRSRYYRQRQRSRRRRRR(COOH). et
RRRLHRIHRRQHRSCRRRKRR(COOH).
pour effectuer le transfert in vitro, et/ou ex vivo d'au moins un acide nucléique, ledit oligonucléotide étant associé ou non à un élément de ciblage.

29. Utilisation d'une composition pharmaceutique selon l'une des revendications 1 à 20 ou 22 pour la fabrication d'un médicament destiné au traitement de maladies résultant d'une déficience en un produit protéique ou nucléique, l'acide nucléique administré codant pour ledit produit protéique ou contenant la séquence correspondant audit produit nucléique.

## Patentansprüche

1. Pharmazeutische Zusammensetzung, die für die Transfektion einer Zelle mit einer Nucleinsäure geeignet ist und außer der Nucleinsäure wenigstens ein Transfektionsmittel und eine Verbindung, die auf die Kondensation (Kompaktierung) der Nucleinsäure einwirkt, umfasst, **dadurch gekennzeichnet, dass** die Verbindung ganz oder teilweise aus Folgendem besteht:
- 2 bis 10 Peptideinheiten (KTPKKAKKP) und/oder (ATPAKKAA), die kontinuierlich wiederholt werden oder auch nicht; oder
- einem Oligopeptid, das ausgewählt ist aus
SRSRYYRQRQRSRRRRRR(COOH) und
RRRLHRIHRRQHRSCRRRICRR(COOH).

2. Pharmazeutische Zusammensetzung gemäß Anspruch 1, **dadurch gekennzeichnet, dass** die Peptideinheiten durch eine oder mehrere Aminosäuren voneinander getrennt sind.

3. Pharmazeutische Zusammensetzung gemäß Anspruch 1 oder 2, **dadurch gekennzeichnet, dass** die Verbindung, die auf die Kondensation der Nucleinsäure einwirkt, ein Oligopeptid ist, das aus (KTPKKAKKP)₂(COOH) und (ATPAKKAA)₂(COOH) ausgewählt ist.

4. Pharmazeutische Zusammensetzung gemäß einem der vorstehenden Ansprüche, **dadurch gekennzeichnet, dass** die Verbindung eine β-Faltblatt-Struktur besitzt.

5. Pharmazeutische Zusammensetzung gemäß einem der Ansprüche 1 bis 4, **dadurch gekennzeichnet, dass** die Verbindung außerdem mit einem Zellrezeptorliganden assoziiert ist.

6. Pharmazeutische Zusammensetzung gemäß Anspruch 5, **dadurch gekennzeichnet, dass** es sich bei der Verbindung um das Peptid PKKKRKV-bAla-(KTPKKAKKP)₂(COOH) handelt.

7. Pharmazeutische Zusammensetzung gemäß einem der Ansprüche 1 bis 6, **dadurch gekennzeichnet, dass** die Verbindung außerdem mit einem Peptid des fusogenen Typs assoziiert ist, das die Transfektion einer Zelle mit der Zusammensetzung begünstigt.

8. Pharmazeutische Zusammensetzung gemäß einem der Ansprüche 1 bis 7, **dadurch gekennzeichnet, dass** die Verbindung außerdem polyglycosyliert, sulfoniert, phosphoryliert und/oder auf komplexe Zucker oder ein lipophiles Agens gepfropft ist.

9. Pharmazeutische Zusammensetzung gemäß einem der vorstehenden Ansprüche, **dadurch gekennzeichnet, dass** das Transfektionsmittel ein kationisches Polymer oder ein Lipofektionsmittel ist.

10. Pharmazeutische Zusammensetzung gemäß Anspruch 9, **dadurch gekennzeichnet, dass** es sich bei dem Lipofektionsmittel um ein Lipid, das Liposomen bilden kann, sterisch stabilisierte Liposomen (stealth liposomes), Immunoliposomen oder zielgesteuerte Liposomen handelt.

11. Pharmazeutische Zusammensetzung gemäß Anspruch 9, **dadurch gekennzeichnet, dass** das kationische Polymer vorzugsweise eine Verbindung der allgemeinen Formel (I) ist: 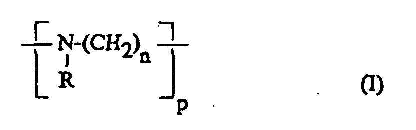 wobei
- R ein Wasserstoffatom ist;
- n eine ganze Zahl zwischen 2 und 10 ist;
- p eine ganze Zahl ist, wobei p so groß ist, dass das mittlere Molekulargewicht des Polymers zwischen 100 und 10⁷ Da liegt.

12. Pharmazeutische Zusammensetzung gemäß Anspruch 9, **dadurch gekennzeichnet, dass** das kationische Polymer aus Polyethylenimin (PEI) und Polypropylenimin (PPI) ausgewählt ist.

13. Pharmazeutische Zusammensetzung gemäß Anspruch 12, **dadurch gekennzeichnet, dass** das Polymer aus Polyethylenimin mit einem mittleren Molekulargewicht von 50 000 Da (PEI50K) und Polyethylenimin mit einem mittleren Molekulargewicht von 800 000 Da (PEI800K) ausgewählt ist.

14. Pharmazeutische Zusammensetzung gemäß Anspruch 9, **dadurch gekennzeichnet, dass** das Lipofektionsmittel wenigstens einen Polyaminbereich umfasst, der durch Spermin oder Thermin repräsentiert wird.

15. Pharmazeutische Zusammensetzung gemäß Anspruch 9, **dadurch gekennzeichnet, dass** das Lipofektionsmittel wenigstens einen lipophilen Bereich umfasst, der durch die allgemeine Formel (IV) 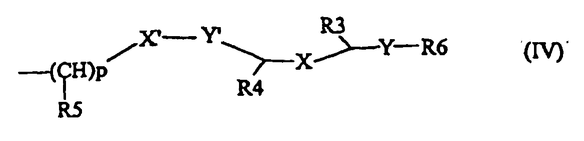 dargestellt wird, wobei
- X und X' unabhängig voneinander ein Sauerstoffatom, eine Methylengruppe -(CH₂)_{q}-, wobei q = 0, 1, 2 oder 3 ist, oder eine Aminogruppe -NH- oder -NR'-, wobei R' eine C₁- bis C₄-Alkylgruppe ist, darstellen;
- Y und Y' unabhängig voneinander eine Methylengruppe, eine Carbonylgruppe oder eine Gruppe C=S darstellen;
- R₃, R₄ und R₅ unabhängig voneinander ein Wasserstoffatom oder einen substituierten oder unsubstituierten C₁- bis C₄-Alkylrest darstellen, wobei p zwischen 0 und 5 variieren kann;
- R₆ ein Cholesterinderivat oder eine Aminoalkylgruppe NR₁R₂ darstellt, wobei R₁ und R₂ unabhängig voneinander einen aliphatischen, gesättigten oder ungesättigten, geradkettigen oder verzweigten C₁₂- bis C₂₂-Rest darstellen.

16. Pharmazeutische Zusammensetzung gemäß Anspruch 9, **dadurch gekennzeichnet, dass** das Lipofektionsmittel ein Lipopolyamin ist, das aus Dioctadecylamidoglycylspermin (DOGS), Palmitoylphosphatidylethanolamin-5-carboxyspermylamid (DPPES), 2,5-Bis(3-aminopropylamino)pentyl(dioctadecylcarbamoylmethoxy)acetat oder 1,3-Bis(3-aminopropylamino)-2-propyl(dioctadecylcarbamoylmethoxy)acetat, {H₂N(CH₂)₃}₂N(CH₂)₄N{(CH₂)₃-NH₂}(CH₂)₃NHCH₂COGlyN[(CH₂)₁₇-CH₃]₂, H₂N(CH₂)₃NH(CH₂)₄NH(CH₂)₃NH-CH₂COGlyN[(CH₂)₁₈]₂ oder H₂N(CH₂)₃NH(CH₂)₄NH(CH₂)₃NHCH₂COArgN-[(CH₂)₁₈] ausgewählt ist.

17. Pharmazeutische Zusammensetzung gemäß einem der Ansprüche 1 bis 9 und 14 bis 16, **dadurch gekennzeichnet, dass** es sich bei dem Transfektionsmittel um Dioctadecylamidoglycylspermin (DOGS) handelt.

18. Pharmazeutische Zusammensetzung gemäß einem der vorstehenden Ansprüche, **dadurch gekennzeichnet, dass** die Nucleinsäure eine Desoxyribonucleinsäure (DNA) ist.

19. Pharmazeutische Zusammensetzung gemäß einem der Ansprüche 1 bis 17, **dadurch gekennzeichnet, dass** die Nucleinsäure eine Ribonucleinsäure (RNA) ist.

20. Pharmazeutische Zusammensetzung gemäß Anspruch 18 oder 19, **dadurch gekennzeichnet, dass** die Nucleinsäure chemisch modifiziert ist.

21. Pharmazeutische Zusammensetzung gemäß Anspruch 18, 19 oder 20, **dadurch gekennzeichnet, dass** die Nucleinsäure eine Antisense-Nucleinsäure ist.

22. Pharmazeutische Zusammensetzung gemäß einem der Ansprüche 18 bis 20, **dadurch gekennzeichnet, dass** die Nucleinsäure ein therapeutisches Gen umfasst.

23. Pharmazeutische Zusammensetzung gemäß einem der vorstehenden Ansprüche, die außerdem ein oder mehrere neutrale Lipide umfasst.

24. Pharmazeutische Zusammensetzung gemäß Anspruch 23, **dadurch gekennzeichnet, dass** das bzw. die neutralen Peptide aus synthetischen oder natürlichen, unter physiologischen Bedingungen zwitterionischen oder ohne Ionenladung vorliegenden Lipiden ausgewählt sind.

25. Pharmazeutische Zusammensetzung gemäß Anspruch 23 oder 24, **dadurch gekennzeichnet, dass** das bzw. die neutralen Peptide aus Dioleoylphosphatidylethanolamin (DOPE), Oleoylpalmitoylphosphatidylethanolamin (POPE), Distearoyl-, -palmitoyl-, -myristoylphosphatidylethanolamin sowie ihren ein- bis dreifach N-methylierten Derivaten, Phosphatidylglycerinen, Diacylglycerinen, Glycosyldiacylglycerinen, Cerebrosiden (wie insbesondere Galactocerebrosiden), Sphingolipiden (wie insbesondere Sphingomyelinen) und Asialogangliosiden (wie insbesondere AsialoGM1 und -GM2) ausgewählt sind.

26. Verwendung einer pharmazeutischen Zusammensetzung gemäß einem der Ansprüche 1 bis 25 für die in-vitro- und/oder ex-vivo-Übertragung von Nucleinsäuren.

27. Verwendung einer Verbindung, die ganz oder teilweise aus Peptideinheiten (KTPKKAKKP) und/oder (ATPAKKAA) besteht, wobei die Zahl dieser Einheiten zwischen 2 und 10 variieren kann, wobei die Verbindung mit einem Liganden eines Zellrezeptors, einem Antikörper oder Antikörperderivat gekoppelt ist, zur in-vitro- und/oder ex-vivo-Zielsteuerung einer Nucleinsäure zu Zellen, die die entsprechenden Rezeptoren oder Antigene exprimieren.

28. Verwendung eines Oligonucleotids, das aus
(ATPAKKAA)₂(COOH),
(KTPKKAKKP)₂(COOH),
ATPKKSAKKTPKKAKKP(COOH),
KKAKSPKKAKAAKPKKAPKSPAKAKA(COOH),
SRSRYYRQRQRSRRRRRR(COOH)
und
RRRLHRIHRRQHRSCRRRKRR(COOH).
ausgewählt ist, zur Durchführung der in-vitro- und/oder ex-vivo-Übertragung wenigstens einer Nucleinsäure, wobei das Oligonucleotid mit einem Zielsteuerungselement assoziiert ist oder auch nicht.

29. Verwendung einer pharmazeutischen Zusammensetzung gemäß einem der Ansprüche 1 bis 20 oder 22 zur Herstellung eines Medikaments zur Behandlung von Krankheiten, die aus dem Mangel an einem Protein- oder Nucleinsäureprodukt resultiert, wobei die verabreichte Nucleinsäure für das Proteinprodukt codiert oder die Sequenz, die dem Nucleinsäureprodukt entspricht, enthält.

## Claims

1. A composition which is useful for the transfection of a nucleic acid, containing apart from said nucleic acid at least one transfection agent and a compound involved in the condensation of said nucleic acid, **characterized in that** said compound is constituted entirely or in part by:
- 2 to 10 (KTPKKAKKP) and/or (ATPAKKAA) peptide units repeated continuously or non-continuously,
- or an oligopeptide chosen from
SRSRYYRQRQRSRRRRRR(COOH) and
RRRLHRIHRRQHRSCRRRKRR(COOH)

2. A pharmaceutical composition according to claim 1, **characterized in that** the peptide units are separated from each other by one or more amino acids.

3. A pharmaceutical composition according to claim 1 or 2, **characterized in that** the compound involved in the condensation of said nucleic acid is an oligopeptide chosen from (KTPKKAKKP)₂(COOH) and (ATPAKKAA)₂(COOH).

4. A pharmaceutical composition according to one of the previous claims, **characterized in that** said compound has a β-sheet structure.

5. A pharmaceutical composition according to one of claims 1 to 4, **characterized in that** said compound is also associated with a cell receptor ligand.

6. A pharmaceutical composition according to claim 5, **characterized in that** said compound is the PKKKRKV-bAla-(KTPKKAKKP)₂(COOH) peptide.

7. A pharmaceutical composition according to one of claims 1 to 6, **characterized in that** said compound is also associated with a peptide of fusogenic type which promotes the cellular transfection of said composition.

8. A pharmaceutical composition according to one of claims 1 to 7, **characterized in that** said compound is also polyglycosylated, sulphonated, phosphorylated and/or grafted to complex sugars or to a lipophilic agent.

9. A pharmaceutical composition according to one of the previous claims, **characterized in that** the transfection agent is a cationic polymer or a lipofectant.

10. A pharmaceutical composition according to claim 9, **characterized in that** the lipofectant is a lipid which is able to form liposomes, furtive liposomes, immunoliposomes or target liposomes.

11. A pharmaceutical composition according to claim 9, **characterized in that** the cationic polymer is preferably a compound of general formula (I): 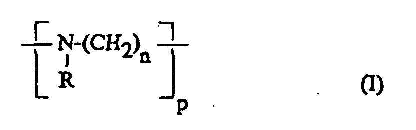 in which
- R is a hydrogen atom
- n is an integer comprised between 2 and 10;
- p is an integer,
with p being such that the average molecular weight of the polymer is comprised between 100 and 10⁷ Da.

12. A pharmaceutical composition according to claim 9, **characterized in that** the cationic polymer is chosen from polyethyleneimine (PEI) and polypropyleneimine (PPI).

13. A pharmaceutical composition according to claim 12, **characterized in that** the polymer is chosen from polyethyleneimine with an average molecular weight of 50,000 Da (PEI50K) and polyethyleneimine with an average molecular weight of 800,000 Da (PEI800K).

14. A pharmaceutical composition according to claim 9, **characterized in that** the lipofectant comprises at least one polyamine region.

15. A pharmaceutical composition according to claim 9, **characterized in that** the lipofectant comprises at least one lipophilic region represented by the general formula (IV) 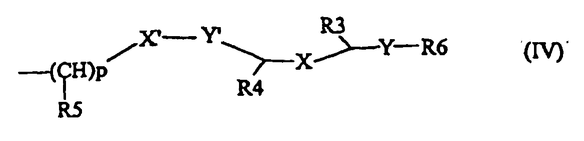 in which
- X and X' represent, independently of each other, an oxygen atom, a methylene group -(CH₂)_{q} - with q equal to 0, 1, 2 or 3, or an amino group
-NH- or -NR'- with R' representing a C1 to C4 alkyl group,
- Y and Y' represent independently of each other a methylene group, a carbonyl group or a C=S group,
- R₃, R₄ and R₅ represent independently of each other a hydrogen atom or a substituted or unsubstituted C₁ to C₄ alkyl radical, with p being able to vary between 0 and 5,
- R₆ represents a cholesterol derivative or an alkylamino group -NR₁ R₂ with R₁ and R₂ representing, independently of each other, a saturated or unsaturated linear or branched C₁₂ to C₂₂ aliphatic radical.

16. A pharmaceutical composition according to claim 9, **characterized in that** the lipofectant is a lipopolyamine chosen from dioctadecylamidoglycylspermine (DOGS), palmitoylphosphatidylethanolamine 5-carboxyspermylamide (DPPES), 2,5-bis(3-aminopropylamino)pentyl (dioctadecylcarbamoylmethoxy)acetate or 1,3-bis(3-aminopropylamino)-2-propyl (dioctadecylcarbamoylmethoxy)acetate, {H₂N(CH₂)₃}₂N(CH₂)₄N{(CH₂)₃NH₂}(CH₂)₃NHCH₂COGlyN[(CH₂)₁₇-CH₃]₂, H₂N(CH₂)₃NH(CH₂)₄NH(CH₂)₃NHCH₂COGlyN[(CH₂)₁₈]₂ or H₂N(CH₂)₃NH(CH₂)₄NH(CH₂)₃NHCH₂COArgN [(CH₂)₁₈].

17. A pharmaceutical composition according to any one of claims 1 to 9 and 14 to 16, **characterized in that** the transfection agent is dioctadecylamidoglycylspermine (DOGS).

18. A pharmaceutical composition according to one of the previous claims, **characterized in that** the nucleic acid is a deoxyribonucleic acid.

19. A pharmaceutical composition according to one of claims 1 to 17, **characterized in that** the nucleic acid is a ribonucleic acid.

20. A pharmaceutical composition according to claim 18 or 19 **characterized in that** the nucleic acid is chemically modified.

21. A pharmaceutical composition according to claim 18, 19 or 20 **characterized in that** the nucleic acid is antisense.

22. A pharmaceutical composition according to one of claims 18 to 20, **characterized in that** the nucleic acid comprises a therapeutic gene.

23. A pharmaceutical composition according to one of the previous claims, which also comprises one or more neutral lipids.

24. A pharmaceutical composition according to claim 23, **characterized in that** the neutral lipid or lipids are chosen from synthetic or natural lipids, zwitterionic lipids or lipids lacking ionic charge under physiological conditions.

25. A pharmaceutical composition according to claim 23 or 24, **characterized in that** the neutral lipid or lipids are chosen from dioleoylphosphatidylethanolamine (DOPE), oleoylpalmitoylphosphatidylethanolamine (POPE), di-stearoyl, palmitoyl-, -myristoyl- phosphatidylethanolamine and their derivatives N-methylated 1 to 3 times, phosphatidylglycerols, diacylglycerols, glycosyldiacylglycerols, cerebrosides, (such as in particular galactocerebrosides), sphingolipids (such as in particular sphingomyelins) and asialogangliosides (such as in particular asialoGM1 and GM2).

26. Use of a pharmaceutical composition according to one of claims 1 to 25, for the in vitro and/or ex vivo transfer of nucleic acids.

27. Use of a compound constituted entirely or in part by peptide units (KTPKKAKKP) and/or (ATPAKKAA) with the number of these units being able to vary between 2 and 10, so that, when it is coupled with a cell receptor ligand, an antibody or an antibody derivative, to target, in vitro and/or ex vivo, a nucleic acid towards cells expressing the corresponding receptors or antigens.

28. Use of an oligopeptide selected from:
(ATPAKKAA)₂(COOH),
(KTPKKAKKP)₂(COOH),
ATPKKSAKKTPKKAKKP(COOH),
KKAKSPKKAKAAKPKKAPKSPAKAKA(COOH),
SRSRYYRQRQRSRRRRRR(COOH) and
RRRLHRIHRRQHRSCRRRKRR(COOH)
in order to carry out the transfer in vitro and/or ex vivo of at least one nucleic acid, said oligonucleotide being associated or not associated with a target element.

29. Use of a pharmaceutical composition according to one of claims 1 to 20 or 22 for the production of a medicament intended for the treatment of diseases resulting from a deficiency in a protein or nucleic product, the administered nucleic acid coding for said protein product or containing the sequence corresponding to said nucleic product.
